# EUROPEAN PATENT SPECIFICATION

(11) **EP 3 852 630 B1**
(45) Date of publication and mention of the grant of the patent: **22.11.2023**
(21) Application number: 19862965.1
(22) Date of filing: 04.09.2019
(51) Int. Cl.: A61B 5/15, A61B 5/151

(54) **IMPROVED ACTIVATION AND RETRACTION MECHANISM FOR LANCING DEVICES**
VERBESSERTER AKTIVIERUNGS- UND RÜCKZUGSMECHANISMUS FÜR LANZETTEN
MÉCANISME D'ACTIVATION ET DE RÉTRACTION AMÉLIORÉ POUR DISPOSITIF AUTOPIQUEUR

(30) Priority: 20.09.2018 SE 1851116
(43) Date of publication of application: 28.07.2021
(73) Proprietor: Safety Lancet Sweden AB, 413 05 Göteborg (SE)
(72) Inventor: AHLBERG, Björn, 112 64 Stockholm (SE); HAMBERG, John, 139 50 Värmdö (SE); SJÖQVIST, Ingvar, 125 73 Älvsjö (SE)
(74) Representative: Bergenstråhle & Partners AB
(86) International application number: PCT/SE2019/050826
(87) International publication number: WO 2020/060465

(56) References cited:
- EP-A1- 1 247 489
- WO-A1-2009/052710
- WO-A1-2018/022535
- WO-A2-2009/129349
- CN-A- 106 491 142
- CN-A- 106 580 340
- CN-A- 107 334 479
- CN-A- 107 951 494
- US-A- 4 924 879
- US-A1- 2007 185 515
- US-A1- 2007 293 882
- US-A1- 2013 123 824
- US-A1- 2013 123 824
- US-B1- 6 231 531

## Description

### Technical field

The present invention relates to an activation/retraction mechanism for lancing devices according to the preamble of claim 1 and a lancing device. Such an activation/retraction mechanism and corresponding lancing device is known from WO 2009/052710 A1.

### Background art

Lancing devices are used to penetrate the skin of a subject and obtain a sample of blood or other body fluid, e.g. in the testing of blood sugar levels by diabetics. Typically, a lancet having a sharp point is translationally mounted within a housing portion of a lancing device. The lancet is driven by a spring or other biasing means to cause the sharp point to extend a small distance through an opening in the housing and into the subject's skin, creating a wound from which the sample of body fluid is collected.

Lancing devices typically are intended either for single-use or for multiple uses. Single-use lancing devices generally are disposed of after one use. For example, in a hospital or clinic, it is desirable to provide a single-use lancing device that can be used on a patient and then disposed of to eliminate any risk of infection to subsequent patients or caregivers from exposure to residual body fluids remaining on the lancing device. Accordingly, single-use lancing devices oftentimes include a disabling mechanism to prevent accidental or unintentional re-use of the device. Various forms of disabling mechanisms are available and are well known in the art. For example, the disabling mechanism may comprise a return spring for retracting the sharp point of the lancet back into the housing after use, as well as break-away elements or a frangible link in the cocking or triggering mechanism to prevent re-arming or re-firing the device after use, a locking element, and/or a shield for blocking travel of the lancet.

One problem experienced by many subjects is that the sharp point of the lancet causes significant pain during penetration. Many lancing devices seek to ease the pain by allowing for adjustment of the penetration depth of the lancet. On the other hand, in order to obtain a sufficient size for a blood sample, the penetration depth should be sufficient to penetrate the epidermis and puncture a capillary.

Another issue affecting the level of pain experienced by the subject is the actual time that the sharp point of the lancet remains in the subject's skin. In lancing devices employing a retraction mechanism including a return spring, the spring force of the activation spring is many times greater than that of the return spring to achieve a sufficiently rapid and powerful lancing stroke. Hence, the sharp point of the lancet remains a considerable period of time in the subject's skin before the return spring is able to overcome the spring force of the activation spring. Examples of such lancing devices are given in US 2013/0123824, US 2007/0185515, US 2007/0293882, EP 1 247 489 A1, and WO 2018/022535 A1.

WO 2009/052710 A1 discloses a safety lancet, wherein a first spring and a second spring are provided at two ends inside a housing respectively, a protective rod is adjacent to an outlet provided on the housing, and a press switch is preset on the housing. A first slide block and a second slide block are arranged between the first spring and the second spring, and a needle is positioned on the end of the second slide block opposite to the outlet. Two pairs of runners extend axially from the opposite end faces of the first slide block and the second slide block, and the two pairs of runners are embedded in a pair of corresponding slide slots within the housing. And the interfaces of the two pairs of slide runners are configured as opposite inclined planes. The inclined planes are brought into contact with each other closely under the force applied by the first spring and the second spring.

US 6,231,531 B1 discloses an apparatus and method for minimizing a subject's pain perception during an invasive medical procedure, such as blood sampling and the like. An invasive sharp can be substantially permanently supported and moveably disposed with respect to a receiving surface such that the invasive sharp reciprocates between a first position in which the sharp is spaced from the receiving surface, away from the subject's skin, and a second position in which at least a portion of the sharp protrudes through an opening in the receiving surface.

Thus, there is a need for a solution which reduces the time that the sharp point of the lancet remains in the subject's skin.

### Summary of the invention

An object of the present invention is to provide an improved activation/retraction mechanism for a lancing device which rapidly returns the lancet into the housing after penetration into the subject's skin. This object is achieved in the present invention, in which there is provided an activation/retraction mechanism for a lancing device according to claim 1 comprising: a pusher comprising a first biasing member arranged to displace the pusher in a distal direction; and a lancet holder comprising a lancet for penetrating the skin of a subject and a second biasing member arranged to displace the lancet holder in a proximal direction; wherein the lancet holder and the pusher are arranged in a housing of the lancing device in such a way that a distal end of the pusher engages a proximal end of the lancet holder to push the lancet holder in a distal direction when the first biasing member displaces the pusher distally, wherein the directions of displacement of the pusher and the lancet holder are substantially parallel to a longitudinal extension of the housing wherein the distal end of the pusher and the proximal end of the lancet holder are arranged to disengage from each other when the pusher has been displaced distally a predetermined length from its initial position, i.e. the length of the firing/lancing stroke, whereby the lancet holder is allowed to return to its initial position under influence of the second biasing member, and wherein an engagement surface of the proximal end of the lancet holder is perpendicular to the displacement direction of the lancet holder.

By providing means for disengaging the pusher and the lancet holder at the end of the firing stroke, the lancet holder may be retracted more rapidly into the housing without needing to overcome the force of the biasing member, thus reducing the time that the lancet remains in the skin.

Preferred embodiments of the present invention are defined in the dependent claims.

In preferred embodiment, a distal portion of the pusher and the proximal portion of the lancet holder have complementary cross-sections and have a combined cross-sectional area smaller than the cross-sectional area of the housing such that the distal portion of the pusher and the proximal portion of the lancet holder are free to move in relation to each other when the distal end of the pusher is disengaged from the proximal end of the lancet holder. Preferably, the cross-sectional area of each of the distal portion of the pusher and the proximal portion of the lancet holder is approximately half of the cross-sectional area of the housing to allow the proximal portion of the lancet holder to pass by the distal portion of the pusher and return to its initial position.

In an advantageous embodiment, the pusher and the lancet holder are arranged at a non-zero angle to each other such that the distal end of the pusher moves laterally in relation to the proximal end of the lancet holder when the first biasing member displaces the pusher distally until they disengage from each other.

In an alternative embodiment, the pusher comprises an external helical groove or ridge arranged to cooperate with a guide projection arranged in the housing to rotate the pusher when the first biasing member displaces the pusher distally, such that the distal end of the pusher rotates in relation to the proximal end of the lancet holder until they disengage from each other.

The above embodiments present different alternatives for displacing the pusher and the lancet holder in relation to each other (lateral movement enabled by the non-zero angle or rotational movement), separate from the firing/lancing displacement in the distal direction, in order to disengage the proximal end of the lancet holder from the distal end of the pusher.

In a further preferred embodiment, the pusher comprises at least one pusher arm extending in a distal direction, wherein a distal end of the at least one pusher arm comprises an engagement surface arranged to engage the proximal end of the lancet holder and a camming surface, wherein the camming surface is arranged to come into contact with an abutment in the housing arranged in a position corresponding to the predetermined length from the initial position of the pusher, and adapted to deflect the at least one pusher arm laterally to disengage the at least one pusher arm from the proximal end of the lancet holder when the pusher is urged against the abutment in the distal direction. Preferably, the pusher comprises two pusher arms. The provision of pusher arm(s) reduces the bulk of the pusher as well as enables using deflection to disengage the pusher arm(s) from the proximal end of the lancet holder.

In an advantageous embodiment, the activation/retraction mechanism further comprises a safety assembly comprising a lancet guard integrally formed with a distal end of the lancet holder by means of a frangible portion adapted to break when the lancet guard is displaced in relation to the lancet holder, e.g. by a twisting and/or pulling motion. The lancet guard provides additional protection against accidental puncture from the exposed sharp point of the lancet. The frangible portions facilitate removal of the lancet guard.

In a preferred embodiment, the lancet holder further comprises one or more projections arranged to cooperate with corresponding recesses in the housing which allow rotation of the lancet holder within a predetermined angular range. The projections allow the lancet holder to be rotated (e.g. by twisting the lancet guard) a predetermined angle and then prevents further rotation. Thus, continued twisting of the lancet guard will break the frangible portions to allow removal of the lancet guard. It is understood that the solution incorporating the lancet guard and twisting for removal may be employed independently in a lancing device, separate from the function of disengaging the pusher from the lancet holder.

In a further preferred embodiment, the safety assembly further comprises a guide rod rigidly connected to or integrally formed with a proximal end of the lancet holder, wherein the pusher comprises a through-going hole adapted to the shape of the guide rod such that rotation of the lancet holder a predetermined angle in relation to the pusher brings the guide rod into alignment with the hole to allow the pusher to be displaced distally along the guide rod. The guide rod provides additional safety in that it prevents accidental firing of the lancing device. It is understood that the solution incorporating the guide rod and cooperating hole in the pusher for preventing accidental firing may be employed independently in a lancing device, separate from the function of disengaging the pusher from the lancet holder.

In an advantageous embodiment, the first and/or second biasing member comprises a resilient spring. A resilient spring may have different characteristics (spring force, length, etc.) adapted to the desired specification of the lancing device.

In an advantageous embodiment, the activation/retraction mechanism further comprises a releasable locking member arranged to retain the pusher and the first biasing member in a prestressed state. The locking member is activated to release the pusher, i.e. launching the firing stroke of the lancing device.

In a further preferred embodiment, the pusher is arranged at a distance from the lancet holder in an initial stage such that the distal end of the pusher is separated from the proximal end of the lancet holder. By letting the pusher travel an initial distance before engaging the lancet holder, a 'hammer' effect is achieved wherein the distal end of the pusher strikes the proximal end of the lancet holder to propel it rapidly in a distal direction, overwhelming the second biasing member. The hammer effect thus increases the speed of the lancing stroke.

According to the present invention, there is also provided a lancing device as defined by claim 13.

### Brief description of drawings

The invention is now described, by way of example, with reference to the accompanying drawings, in which:
Figs. 1A-1D show cross-sectional views of an activation/retraction mechanism according to a first embodiment of the present invention in different stages of operation;
Figs. 2A-2E show views of an activation/retraction mechanism according to a second embodiment of the present invention in different stages of operation;
Figs. 3A-3D show views of an activation/retraction mechanism according to a third embodiment of the present invention in different stages of operation;
Fig. 4 shows a perspective view of an alternative activation/retraction mechanism according to a third embodiment of the present invention;
Figs. 5A-5F show views of an activation/retraction mechanism according to Fig. 4 in different stages of operation;
Figs. 6A-6D show views of an alternative activation/retraction mechanism which does not form part of the present invention in different stages of operation.

### Detailed description of embodiments

In the following, a detailed description of an activation/retraction mechanism for a lancing device according to the invention is presented. In the drawing figures, like reference numerals designate identical or corresponding elements throughout the several figures. It will be appreciated that these fig-ures are for illustration only and are not in any way restricting the scope of the invention.

It is understood that in the context of the present invention, the term 'distal' shall be interpreted as referring to a direction away from or portion furthest from the operator handling the lancing device. Likewise, the term `proximal' shall be interpreted as referring to a direction towards or closest to the operator handling the lancing device. In cases where the operator is the same as the subject, e.g. diabetes patients using the lancing device on themselves, the terms shall be interpreted from the perspective of the operator, regardless of the subject.

As explained above, the present invention aims at minimising the pain experienced by a subject when penetrating the skin using a lancing device to obtain a blood sample. The overall concept common to all embodiments described below is that the component (pusher) urging the lancet forward is decoupled from the component (holder) holding the lancet at the end of the firing stroke to allow a more rapid retraction of the lancet from the skin.

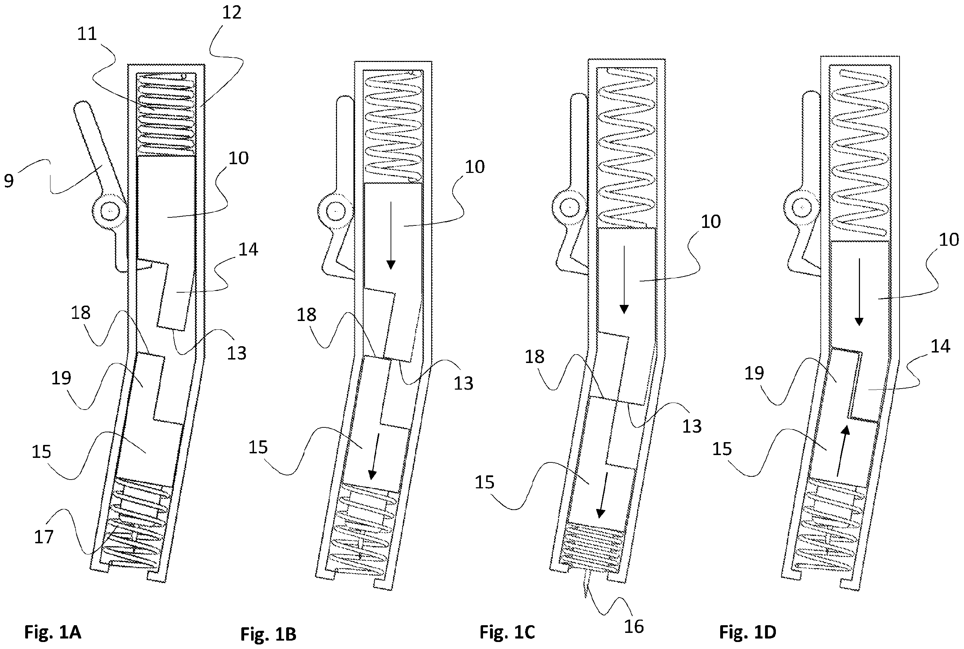

Figs. 1A-1D show a first embodiment of an activation/retraction mechanism for a lancing device according to the present invention during different stages of operation. Generally, the activation/retraction mechanism comprises a pusher 10 adapted to be displaced distally by means of a first biasing member 11 and a lancet holder 15 comprising a lancet 16 and adapted to be engaged and displaced distally by the pusher 10. Further, the lancet holder 15 is arranged to be displaced in a proximal direction by a second biasing member 17. The pusher 10 including the first biasing member 11, and the lancet holder 15 including the lancet 16 and the second biasing member 17 are arranged in a housing. Figs. 1A-1D show the internal space of the housing and the components arranged therein.

Fig. 1A shows the activation/retraction mechanism in an initial stage (before firing) wherein the pusher 10 is retained in a proximal position by a releasable locking member 9. The first biasing member 11 is here compressed in a prestressed state. The locking member 9 is here shown as a lever with a catch engaging the pusher 10 and is arranged to pivot about an axis to release the pusher 10. Other variants of the locking member 9 are also envisaged.

The lancet holder 15 is arranged in a distal portion of the housing and comprises a lancet 16 at a distal end thereof. The lancet holder 15 is biased proximally towards a retracted position, in which the lancet 16 is accommodated in the housing without protruding therefrom, by means of a second biasing member 17. The second biasing member 17 is here in a relaxed state.

As may be seen, the distal portion of the housing, wherein the lancet holder 15 is arranged, is angled with respect to the proximal portion of the housing, wherein the pusher 10 is arranged. Furthermore, the distal portion 14 of the pusher 10 is oriented at an angle to the longitudinal extension of the pusher 10, which angle substantially corresponds to the angle of the distal portion of the housing with respect to the proximal portion. Moreover, the shape and/or cross-section of the distal portion 14 of the pusher 10 corresponds to the shape and/or cross-section of the proximal portion 19 of the lancet holder 15. For instance, both the pusher 10 and the lancet holder 15 may have generally cylindrical shapes, whereas the distal portion 14 of the pusher 10 and the proximal portion 19 of the lancet holder 15 may both have the shape of a half-cylinder. Other complementary shapes are also contemplated which fulfil the criteria that the distal portion 14 of the pusher 10 and the proximal portion 19 of the lancet holder 15 are free to move in relation to each other in a distal and proximal direction, respectively, at the end of the firing stroke of the pusher 10, i.e. when the proximal end 18 of the lancet holder 15 is disengaged from the distal end 13 of the pusher 10.

In Fig. 1B, the locking member 9 has been actuated and the pusher 10 has been released to move in a distal direction under influence of the first biasing member 11. After travelling a short distance, the distal end 13 of the pusher 10 reaches and abuts against the proximal end 18 of the lancet holder 15. Further displacement of the pusher 10 thus also displaces the lancet holder 15 in a distal direction. By letting the pusher 10 travel an initial distance before engaging the lancet holder 15, a 'hammer' effect is achieved wherein the distal end 13 of the pusher 10 strikes the proximal end 18 of the lancet holder 15 to propel it rapidly in a distal direction, overwhelming the second biasing member 17. Of course, the pusher 10 and the lancet holder 15 may be arranged such that the distal end 13 of the pusher 10 engages the proximal end 18 of the lancet holder 15 already in the initial state of the activation/retraction mechanism.

In Fig. 1C, at or near the end of the firing stroke, the pusher 10 has almost reached its distalmost position. The lancet holder 15 has also reached its distalmost position wherein the lancet 16 extends out of the distal opening of the housing to penetrate the skin, and the second biasing member 17 has been compressed. As may be seen in Fig. 1C, the pusher 10 has been displaced distally, but the distal portion 14 of the pusher 10 has also been displaced laterally in relation to the proximal portion 19 of the lancet holder 15. Simultaneously with the distal displacement, the distal end 13 of the pusher 10 slides laterally with respect to the proximal end 18 of the lancet holder 15 until their respective end surfaces lose contact with each other, and as a result, the proximal end 18 of the lancet holder 15 is disengaged from the distal end 13 of the pusher 10.

Fig. 1D shows the activation/retraction mechanism in a final stage, wherein the pusher 10 has been displaced to its distalmost position. Due to the complementary shapes of the distal portion 14 of the pusher 10 and the proximal portion 19 of the lancet holder 15, the lancet holder 15 is allowed to be displaced by the second biasing member 17 in a proximal direction towards its initial, retracted position, wherein the lancet 16 does not protrude from the housing. Each of the distal portion 14 of the pusher 10 and the proximal portion 19 of the lancet holder 15, respectively, comprises a recess adapted to accommodate the corresponding protruding portion of the other component.

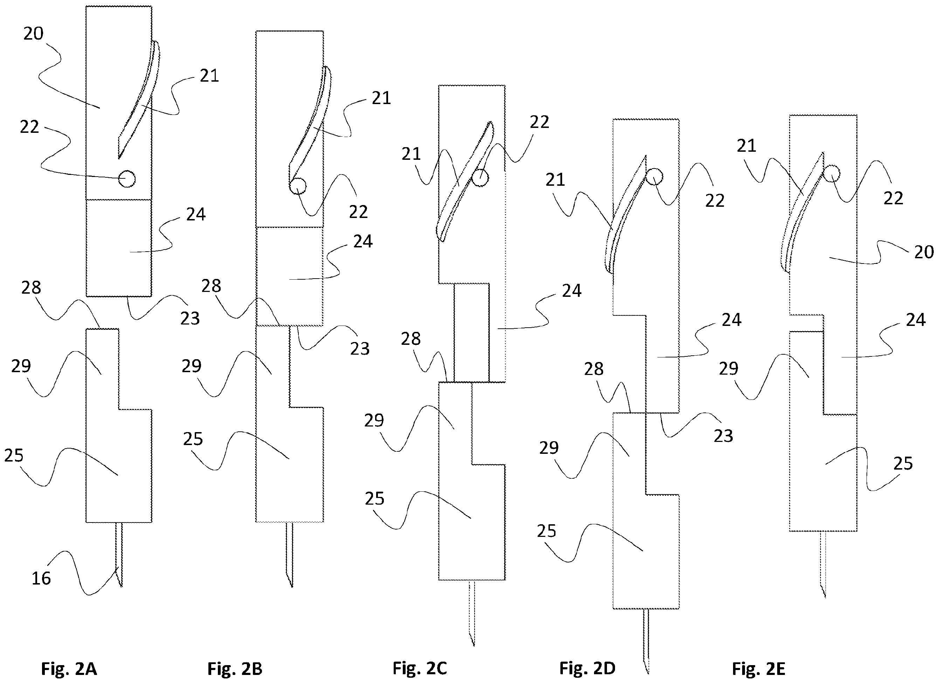

Turning now to Figs. 2A-2E, an alternative embodiment of the activation/retraction mechanism working under a similar principle to the one shown in Figs. 1A-1D is illustrated. This alternative activation/retraction mechanism provides relative movement of the pusher 20 and the lancet holder 25, separate from the distal displacement. In Figs. 2A-2E, the housing, the locking member, and the biasing members acting on the components have been omitted so as not to clutter the drawings. However, it should be understood that said components are present in conjunction with the activation/retraction mechanism as a whole.

Fig. 2A shows the activation/retraction mechanism in an initial stage (before firing) wherein the pusher 20 is retained in a proximal position, and the lancet holder 25 is biased towards its proximal position with the lancet not protruding from the housing. The pusher 20 comprises a helical ridge 21 or guide groove 21 extending along part of its circumferential surface. The ridge 21 is arranged to cooperate with a projection 22 arranged on the internal surface of the housing, as will be further explained below.

Fig. 2B shows the activation/retraction mechanism shortly after firing, the pusher 20 has been released and displaced distally by the first biasing member so that the distal end 23 of the pusher 20 abuts against the proximal end 28 of the lancet holder 25. Simultaneously, the ridge 21 has been brought into engagement with the projection 22 on the housing.

Fig. 2C shows the activation/retraction mechanism in a further advanced stage, wherein the pusher 20 and the lancet holder 25 both have been displaced further in a distal direction owing to the abutment between the distal end 23 of the pusher 20 and the proximal end 28 of the lancet holder 25. At the same time, the helical path of the ridge 21 slides against the projection 22 to cause the pusher 20 to rotate in relation to the housing and the lancet holder 25. As a result, the area of contact between the distal end 23 of the pusher 20 and the proximal end 28 of the lancet holder 25 decreases.

Fig. 2D shows the activation/retraction mechanism at or near the end of the firing stroke. The pusher 20 has reached its distalmost position and has also been rotated a quarter-turn, approximately 90° about the longitudinal axis of the pusher 20. The lancet holder 25 has also reached its distalmost position, wherein the lancet extends out of the distal opening of the housing to penetrate the skin. At this point, the area of contact between the distal end 23 of the pusher 20 and the proximal end 28 of the lancet holder 25 reaches its minimum and becomes zero, in other words, the proximal end 28 of the lancet holder 25 is disengaged from the distal end 23 of the pusher 20.

Fig. 2E shows the activation/retraction mechanism in a final stage. Due to the complementary shapes of the distal portion 24 of the pusher 20 and the proximal portion 29 of the lancet holder 25, the lancet holder 25 is allowed to be displaced by the second biasing member in a proximal direction towards its initial, retracted position, wherein the lancet does not protrude from the housing. Each of the distal portion 24 of the pusher 20 and the proximal portion 29 of the lancet holder 25, respectively, comprises one or more recesses adapted to accommodate the corresponding protruding portions of the other component. For instance, the distal portion 24 of the pusher 20 may comprise one or more protruding members and the proximal portion 29 of the lancet holder 25 may comprise correspondingly shaped recesses, or vice versa. As the pusher 20 is rotated by the action of the projection 22 and the helical ridge 21, the protruding members are brought into alignment with the recesses. When aligned, the protruding members will be received in the recesses as the lancet holder 25 is displaced in the proximal direction.

Similar to the embodiment described with reference to Figs. 1A-1D, the shape and/or cross-section of the distal portion 24 of the pusher 20 and the proximal portion 29 of the lancet holder 25 are adapted to correspond to each other in such a way to allow free movement of the lancet holder 25 in a proximal direction in relation to the pusher 20 after disengagement. In Figs. 2A-2E, the distal portion 24 of the pusher 20 and the proximal portion 29 of the lancet holder 25 are illustrated as having the shape of a half-cylinder. Other shapes or cross-sections are also contemplated, e.g. one or more recesses adapted to accommodate one or more corresponding protruding portions.

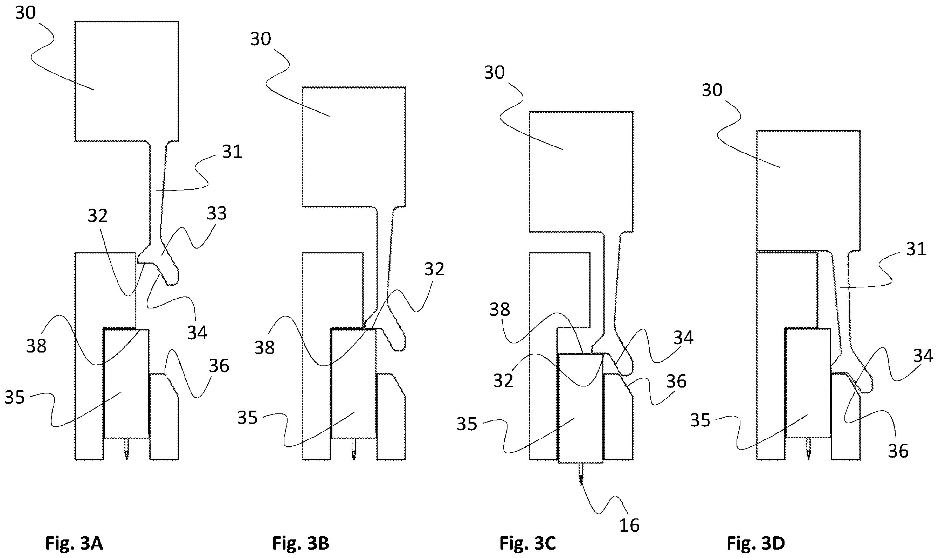

Turning now to Figs. 3A-3D, another embodiment of the activation/retraction mechanism is here illustrated. Again, the housing, the locking member, and the biasing members acting on the components have been omitted so as not to clutter the drawings. In this embodiment, the pusher 30 is provided with a flexible pusher arm 31 extending in a distal direction towards the lancet holder 35. A distal end 33 of the at least one pusher arm 31 comprises an engagement surface 32 arranged to engage the proximal end 38 of the lancet holder 35 and a camming surface 34. The camming surface 34 is arranged to come into contact with an abutment 36 in the housing arranged in a position corresponding to the predetermined length from the initial position of the pusher 30, and adapted to deflect the at least one pusher arm 31 laterally to disengage the at least one pusher arm 31 from the proximal end 38 of the lancet holder 35 when the pusher 30 is urged against the abutment 36 in the distal direction.

Fig. 3A shows the activation/retraction mechanism in an initial stage (before firing) wherein the pusher 30 is retained in a proximal position, and the lancet holder 35 is biased towards its proximal position with the lancet not protruding from the housing.

Fig. 3B shows the activation/retraction mechanism shortly after firing, the pusher 30 has been released and displaced distally by the first biasing member so that the engagement surface 32 on the distal end 33 of the pusher arm 31 abuts against the proximal end 38 of the lancet holder 35. Further displacement of the pusher 30 in the distal direction will also displace the lancet holder 35. The engagement surface 32 is preferably planar and faces towards the lancet holder 35 in the distal direction.

Fig. 3C shows the activation/retraction mechanism in a further advanced stage, wherein the pusher 30 and the lancet holder 35 both have been displaced further in the distal direction owing to the abutment 36 between the engagement surface 32 on the distal end 33 of the pusher arm 31 and the proximal end 38 of the lancet holder 35. In this position, the camming surface 34 on the distal end 33 of the pusher arm 31 is brought into contact with the abutment 36 in the housing. As may be seen, the abutment 36 may also comprise a corresponding camming surface 34. Further displacement of the pusher 30 in the distal direction will urge the camming surface 34 on the distal end 33 of the pusher arm 31 against the abutment 36 and cause it to be deflected laterally, i.e. away from the lancet holder 35, until the pusher arm 31 is disengaged from the lancet holder 35.

Fig. 3D shows the activation/retraction mechanism in a final stage at the end of the firing stroke. After the disengagement from the pusher arm 31, the lancet holder 35 is allowed to be displaced by the second biasing member in a proximal direction towards its initial, retracted position, wherein the lancet 16 does not protrude from the housing.

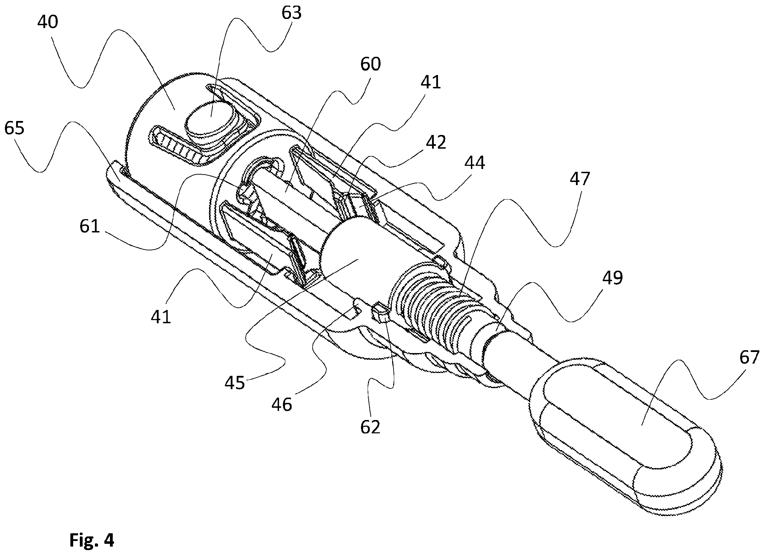

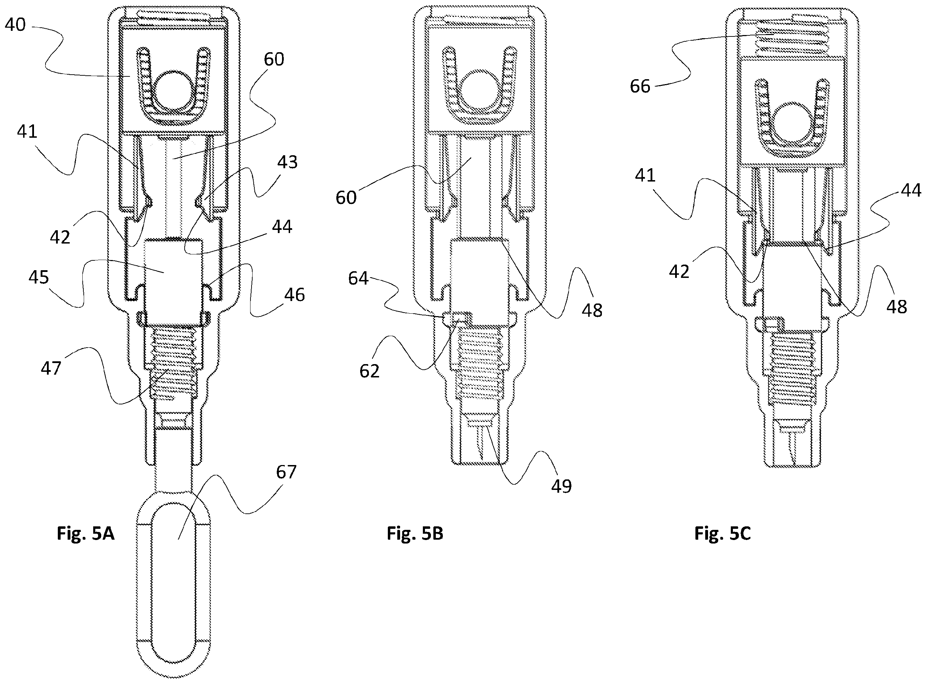

Turning now to Fig. 4 and Figs. 5A-5F, an alternative embodiment of the activation/retraction mechanism working under a similar principle to the one shown in Figs. 3A-3D is illustrated. The activation/retraction mechanism comprises a pusher 40 adapted to be displaced distally by means of a first biasing member 66 and a lancet holder 45 comprising a lancet 16 and adapted to be engaged and displaced distally by the pusher 40. Further, the lancet holder 45 is arranged to be displaced in a proximal direction by a second biasing member 47. The pusher 40 including the first biasing member 66, and the lancet holder 45 including the lancet 16 and the second biasing member 47 are arranged in a housing 65. Fig. 4 and Figs. 5A-5F show the internal space of the housing 65 in a cutaway view and the components arranged therein.

In this embodiment, the pusher 40 comprises two oppositely arranged, flexible pusher arms 41 extending distally, each comprising an engagement surface 42 and a camming surface 44 on a distal end 43 thereof. Similar to the embodiment of Figs. 3A-3D, the housing 65 comprises corresponding abutments 46, with which the camming surfaces 44 are arranged to be brought into contact. Furthermore, the activation/retraction mechanism as illustrated in Fig. 4 comprises additional safety features to prevent accidental firing of the lancing device and protect against injury from the sharp point of the lancet 16.

Firstly, a lancet or needle guard is provided at the distal end 43. The lancet guard 67 may be integrally formed with a distal end 49 of the lancet holder 45 by means of a frangible portion adapted to break when the lancet guard 67 is displaced in relation to the lancet 16. For instance, the lancet guard 67 is adapted to break off from the lancet holder 45 by twisting or pulling the lancet guard 67 away. Additionally, the lancet holder 45 may comprise lateral projections 62 which cooperate with corresponding recesses 64 in the housing 65 which allow rotation of the lancet holder 45 within a predetermined angular range. Hence, when the lancet guard 67 is twisted, the lancet holder 45 will be rotated until the lateral projections 62 abut against end surfaces of the recesses 64 in the housing 65 to prevent further rotation. When the operator twists the lancet guard 67 further, the applied torque will break the frangible portions such that the lancet guard 67 may be removed, and the activation/retraction mechanism will be ready for use.

Fig. 4 also shows a second safety feature of the activation/retraction mechanism which prevents accidental firing of the lancing device. To that end, the lancet holder 45 comprises a guide rod 60 rigidly connected to, or integrally formed with, a proximal end 48 of the lancet holder 45 and extending proximally towards the pusher 40. The cross-section of the guide rod 60 preferably exhibits rotational symmetry of order 2 with respect to its longitudinal axis, i.e. the shape of the guide rod 60 is the same when rotated 180° but varies in the range 0-180°. One such example is given in Fig. 4, wherein the guide rod 60 is shaped like a flat bar.

The pusher 40 in this embodiment is hollow and the first biasing member 66 is arranged in an internal space of the pusher 40. The pusher 40 also comprises a through-going hole 61 adapted to the cross-sectional shape of the guide rod 60. However, in an initial position of the guide rod 60, the hole 61 in the pusher 40 is arranged misaligned with respect to the guide rod 60 such that distal movement of the pusher 40 is prevented. Rotation of the lancet holder 45 a predetermined angle, for instance 45°, in relation to the pusher 40 brings the guide rod 60 into alignment with the hole 61, which allows the guide rod 60 to enter through the hole 61 and the pusher 40 to be displaced distally along the guide rod 60.

It is understood that the features of the safety assembly (lancet guard, guide rod and hole) may be employed in a lancing device independently of the activation/retraction mechanism of the present invention.

Similar to the locking member described in relation to Figs. 1A-1D, the activation/retraction mechanism comprises a depressible button 63 arranged on a flexible flap which is formed by a recess in the pusher 40. In an initial stage, the button 63 is seated in a hole 61 (not shown) in the housing 65 65. Depressing the button 63 releases the pusher 40 from the housing 65 to initiate the firing stroke.

Fig. 5A shows the activation/retraction mechanism of Fig. 4 in an initial stage (before firing) wherein the pusher 40 is retained in a proximal position, and the lancet holder 45 is biased towards its proximal position. Also, the lancet guard 67 is in place covering the distal sharp point of the lancet 16.

Fig. 5B shows the activation/retraction mechanism of Fig. 4 in a ready state, wherein the lancet guard 67 has been removed by twisting it off, which causes the lancet holder 45 and guide rod 60 to be rotated about a predetermined angle until the lateral projections 62 have reached and abut against the end surfaces of the corresponding recesses 64 (not shown) in the housing 65. The rotation brings the guide rod 60 into alignment with the hole 61 in the pusher 40 to allow the pusher 40 to be displaced distally through a sliding motion on the guide rod 60.

Fig. 5C shows the activation/retraction mechanism shortly after firing, the pusher 40 has been released and displaced distally by the first biasing member 66 so that the engagement surfaces 42 on the distal end 43 of each pusher arm 41 abut against the proximal end 48 of the lancet holder 45. Further displacement of the pusher 40 in the distal direction will also displace the lancet holder 45. The engagement surfaces 42 are preferably planar and face towards the lancet holder 45 in the distal direction.

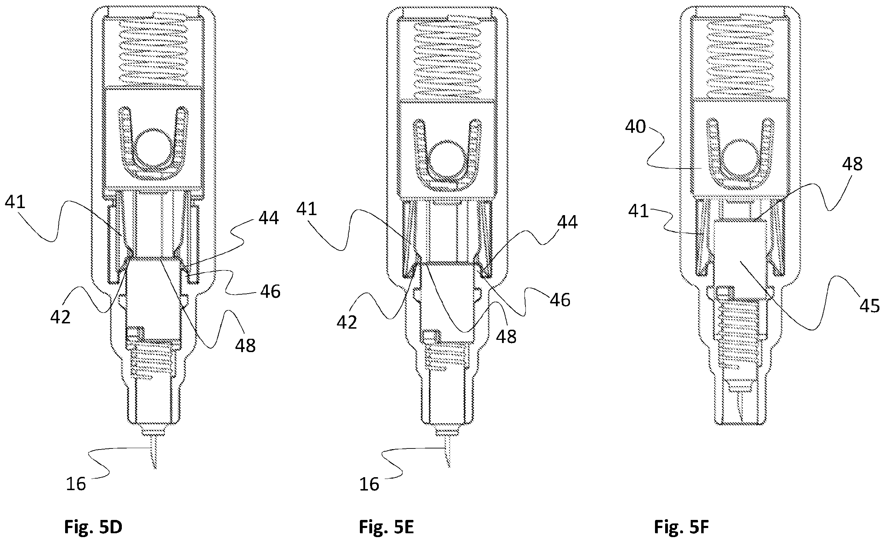

Fig. 5D shows the activation/retraction mechanism in a further advanced stage, wherein the pusher 40 and the lancet holder 45 both have been displaced further in the distal direction owing to the abutment between the engagement surfaces 42 on the distal end 43 of each pusher arm 41 and the proximal end 48 of the lancet holder 45. The lancet 16 now protrudes out of the housing 65 to penetrate the skin of the subject. In this position, the camming surfaces 44 on the distal end 43 of each pusher arm 41 are brought into contact with the corresponding abutments 46 in the housing 65.

Fig. 5E shows the activation/retraction mechanism at or near the end of the firing stroke. Further displacement of the pusher 40 in the distal direction has urged the camming surfaces 44 on the distal end 43 of each pusher arm 41 against the abutments 46 and caused them to deflect laterally, i.e. away from the lancet holder 45, such that the pusher arms 41 are disengaged from the lancet holder 45.

Fig. 5F shows the activation/retraction mechanism in a final stage at the end of the firing stroke. After the disengagement from the pusher arms 41, the lancet holder 45 is free to be displaced by the second biasing member 47 in a proximal direction towards its initial, retracted position, wherein the lancet 16 does not protrude from the housing 65. The first biasing member 66 continues to bias the pusher 40 distally, thus maintaining the pusher 40 in the distal position with the pusher arms 41 laterally deflected.

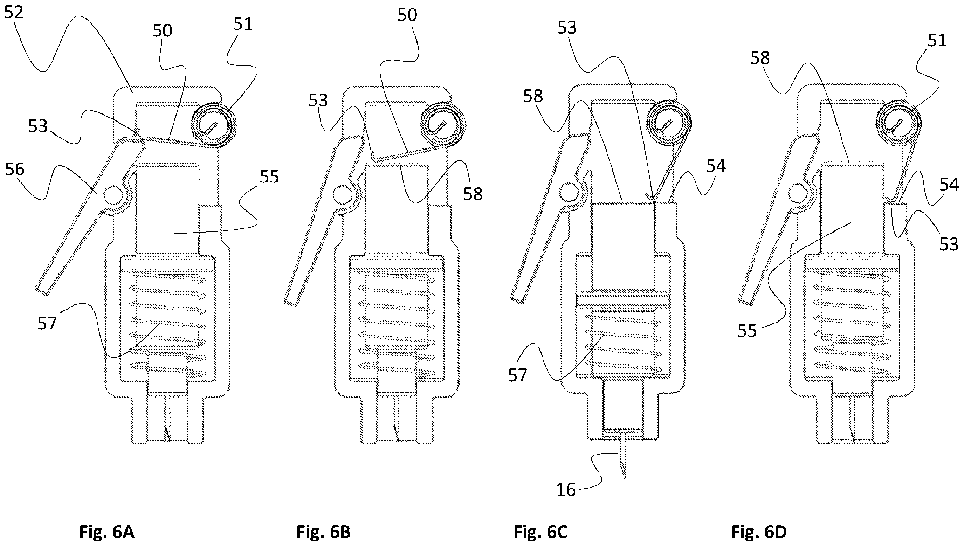

Turning now to Figs. 6A-6D, an alternative activation/retraction mechanism is here illustrated. In this example, the first biasing member acts as the pusher. The first biasing member comprises a spiral wound torsion spring arranged in a proximal end of the housing with its axis of rotation substantially perpendicular to the longitudinal extension of the housing. A free end of the torsion spring which extends a predetermined length from the axis of rotation is arranged to act as the pusher, wherein rotation of the torsion spring causes the free end to engage the proximal end of the lancet holder and displace the lancet holder distally. Furthermore, the housing comprises a recess in the wall thereof arranged to receive the free end of the torsion spring when the free end reaches its distalmost position. The recess is dimensioned and positioned such that the free end is moved sufficiently in a lateral direction to be disengaged from the proximal end of the lancet holder. The lancet holder is thereby free to return to its initial, retracted position under influence of the second biasing member.

Fig. 6A shows the activation/retraction mechanism in an initial stage (before firing) wherein the free end of the torsion spring is retained in a proximal position by a releasable locking member. The first biasing member is here in a prestressed state, wound in a clockwise direction, and oriented substantially perpendicular to the longitudinal extension of the housing (i.e. in the 9 o'clock position). The locking member is here shown as a lever with a catch engaging the free end of the torsion spring and is arranged to pivot about an axis to release the free end. Other variants of the locking member are also envisaged.

In Fig. 6B, the locking member has been actuated and the free end of the torsion spring has been released to rotate about the axis of rotation of the torsion spring in an anti-clockwise direction due to unwinding of the torsion spring. The anti-clockwise rotation also causes the free end to be displaced in a distal direction. After travelling a short distance, the free end of the torsion spring reaches and abuts against the proximal end of the lancet holder. Further displacement of the free end of the torsion spring thus also displaces the lancet holder in a distal direction.

In Fig. 6C, at or near the end of the firing stroke, the free end of the torsion spring has been rotated towards its distalmost position, approximately between the 6 and 7 o'clock position. The lancet holder has also reached its distalmost position wherein the lancet extends out of the distal opening of the housing to penetrate the skin, and the second biasing member has been compressed. During rotation, the free end of the torsion spring slides laterally along the surface of the proximal end of the lancet holder such that the contact point between them gradually shifts from left to right, as illustrated in Figs. 6B and 6C.

Fig. 6D shows the activation/retraction mechanism in a final stage, wherein the free end of the torsion spring has been rotated further to be received in the recess arranged in the wall of the housing. When the free end of the torsion spring enters the recess, it loses contact with the proximal end of the lancet holder, and as a result, the proximal end of the lancet holder is disengaged from the free end of the torsion spring. The lancet holder is then free to be displaced by the second biasing member in a proximal direction towards its initial, retracted position, wherein the lancet does not protrude from the housing.

The first biasing member and/or the second biasing member preferably comprise a resilient spring, such as a helical coil spring. However, other means for biasing are also contemplated, such as for instance a flat coil spring or a spring steel plate.

## Claims

1. An activation/retraction mechanism for a lancing device comprising:
- a pusher (10; 20; 30; 40; 50) comprising a first biasing member (11; 66; 51) arranged to displace the pusher (10; 20; 30; 40; 50) in a distal direction; and
- a lancet holder (15; 25; 35; 45; 55) comprising a lancet (16) for penetrating the skin of a subject, and a second biasing member (17; 47; 57) arranged to displace the lancet holder (15; 25; 35; 45; 55) in a proximal direction;
wherein the lancet holder (15; 25; 35; 45; 55) and the pusher (10; 20; 30; 40; 50) are arranged in a housing (12; 42) of the lancing device in such a way that a distal end (13; 23; 33; 43; 53) of the pusher (10; 20; 30; 40; 50) engages a proximal end (18; 28; 38; 48; 58) of the lancet holder (15; 25; 35; 45; 55) to push the lancet holder (15; 25; 35; 45; 55) in a distal direction when the first biasing member (11; 66; 51) displaces the pusher (10; 20; 30; 40; 50) distally,
wherein the directions of displacement of the pusher (10; 20; 30; 40; 50) and the lancet holder (15; 25; 35; 45; 55) are substantially parallel to a longitudinal extension of the housing (12; 42),
**characterised in that** the distal end (13; 23; 33; 43; 53) of the pusher (10; 20; 30; 40; 50) and the proximal end (18; 28; 38; 48; 58) of the lancet holder (15; 25; 35; 45; 55) are arranged to disengage from each other when the pusher (10; 20; 30; 40; 50) has been displaced distally a predetermined length from its initial position, whereby the lancet holder (15; 25; 35; 45; 55) is allowed to return to its initial position under influence of the second biasing member (17; 47; 57), and
an engagement surface of the proximal end (18; 28; 38; 48; 58) of the lancet holder (15; 25; 35; 45; 55) is perpendicular to the displacement direction of the lancet holder (15; 25; 35; 45; 55).

2. The activation/retraction mechanism according to claim 1, wherein a distal portion (14; 24) of the pusher (10; 20) and a proximal portion (19; 29) of the lancet holder (15; 25) have complementary cross-sections and have a combined cross-sectional area smaller than the cross-sectional area of the housing such that the distal portion (14; 24) of the pusher (10; 20) and the proximal portion (19; 29) of the lancet holder (15; 25) are free to move in relation to each other when the distal end (13; 23) of the pusher (10; 20) is disengaged from the proximal end (18; 28) of the lancet holder (15; 25).

3. The activation/retraction mechanism according to claim 2, wherein the pusher (10) and the lancet holder (15) are arranged at a non-zero angle to each other such that the distal end (13) of the pusher (10) moves laterally in relation to the proximal end (18) of the lancet holder (15) when the first biasing member (17) displaces the pusher (10) distally until they disengage from each other.

4. The activation/retraction mechanism according to claim 2, wherein the pusher (20) comprises an external helical groove or ridge (21) arranged to cooperate with a guide projection (22) arranged in the housing to rotate the pusher (20) when the first biasing member displaces the pusher (20) distally, such that the distal end (23) of the pusher (20) rotates in relation to the proximal end (28) of the lancet holder (25) until they disengage from each other.

5. The activation/retraction mechanism according to claim 1, wherein the pusher (30; 40) comprises at least one pusher arm (31; 41) extending in a distal direction, wherein a distal end (33; 43) of the at least one pusher arm (31; 41) comprises an engagement surface (32; 42) arranged to engage the proximal end (38; 48) of the lancet holder (35; 45) and a camming surface (34; 44), wherein the camming surface (34; 44) is arranged to come into contact with an abutment (36; 46) in the housing arranged in a position corresponding to the predetermined length from the initial position of the pusher (30; 40) and adapted to deflect the at least one pusher arm (31; 41) laterally to disengage the at least one pusher arm (31; 41) from the proximal end (38; 48) of the lancet holder (35; 45) when the pusher (30; 40) is urged against the abutment (36; 46) in the distal direction.

6. The activation/retraction mechanism according to claim 5, wherein the pusher (40) comprises two pusher arms (41).

7. The activation/retraction mechanism according to claim 5 or 6, further comprising a safety assembly comprising a lancet guard (67) integrally formed with a distal end (49) of the lancet holder (45) by means of a frangible portion adapted to break when the lancet guard (67) is displaced in relation to the lancet holder (45).

8. The activation/retraction mechanism according to claim 7, wherein the lancet holder (45) further comprises one or more projections (62) arranged to cooperate with corresponding recesses (64) in the housing (65) which allow rotation of the lancet holder (45) within a predetermined angular range.

9. The activation/retraction mechanism according to claim 7 or 8, wherein the safety assembly further comprises a guide rod (60) rigidly connected to or integrally formed with a proximal end (48) of the lancet holder (45), wherein the pusher (40) comprises a through-going hole (61) adapted to the shape of the guide rod (60) such that rotation of the lancet holder (45) a predetermined angle in relation to the pusher (40) brings the guide rod (60) into alignment with the hole (61) to allow the pusher (40) to be displaced distally along the guide rod (60).

10. The activation/retraction mechanism according to any one of the preceding claims, wherein the first biasing member (11; 66; 51) and/or second biasing member (17; 47; 57) comprises a resilient spring.

11. The activation/retraction mechanism according to any one of the preceding claims, further comprising a releasable locking member (9; 63; 56) arranged to retain the pusher (10; 40; 50) and the first biasing member (11; 66; 51) in a prestressed state.

12. The activation/retraction mechanism according to any one of the preceding claims, wherein the pusher (10; 20; 30; 40; 50) is arranged at a distance from the lancet holder (15; 25; 35; 45; 55) in an initial stage such that the distal end of the pusher (10; 20; 30; 40; 50) is separated from the proximal end (18; 28; 38; 48; 58) of the lancet holder (15; 25; 35; 45; 55).

13. A lancing device comprising an activation/retraction mechanism according to any one of the preceding claims.

## Patentansprüche

1. Aktivierungs-/Einzugsmechanismus für eine Lanzettiervorrichtung, umfassend:
- einen Drücker (10; 20; 30; 40; 50), der ein erstes Vorspannelement (11; 66; 51) umfasst, das angeordnet ist, um den Drücker (10; 20; 30; 40; 50) in eine distale Richtung zu verschieben; und
- einen Lanzettenhalter (15; 25; 35; 45; 55), der eine Lanzette (16) zum Eindringen in die Haut eines Patienten und ein zweites Vorspannelement (17; 47; 57) umfasst, das angeordnet ist, um den Lanzettenhalter (15; 25; 35; 45; 55) in eine proximale Richtung zu verschieben;
wobei der Lanzettenhalter (15; 25; 35; 45; 55) und der Drücker (10; 20; 30; 40; 50) derart in einem Gehäuse (12; 42) der Lanzettiervorrichtung angeordnet sind, dass ein distales Ende (13; 23; 33; 43; 53) des Drückers (10; 20; 30; 40; 50) in ein proximales Ende (18; 28; 38; 48; 58) des Lanzettenhalters (15; 25; 35; 45; 55) eingreift, um den Lanzettenhalter (15; 25; 35; 45; 55) in eine distale Richtung zu drücken, wenn das erste Vorspannelement (11; 66; 51) den Drücker (10; 20; 30; 40; 50) distal verschiebt,
wobei die Verschiebungsrichtungen des Drückers (10; 20; 30; 40; 50) und des Lanzettenhalters (15; 25; 35; 45; 55) im Wesentlichen parallel zu einer Längserstreckung des Gehäuses (12; 42) verlaufen,
**dadurch gekennzeichnet, dass** das distale Ende (13; 23; 33; 43; 53) des Drückers (10; 20; 30; 40; 50) und das proximale Ende (18; 28; 38; 48; 58) des Lanzettenhalters (15; 25; 35; 45; 55) angeordnet sind, um sich voneinander zu lösen, wenn der Drücker (10; 20; 30; 40; 50) um eine vorbestimmte Länge aus seiner Ausgangsposition distal verschoben worden ist, wodurch der Lanzettenhalter (15; 25; 35; 45; 55) unter dem Einfluss des zweiten Vorspannelements (17; 47; 57) in seine Ausgangsposition zurückzukehren erlaubt ist, und
eine Eingriffsfläche des proximalen Endes (18; 28; 38; 48; 58) des Lanzettenhalters (15; 25; 35; 45; 55) senkrecht zur Verschiebungsrichtung des Lanzettenhalters (15; 25; 35; 45; 55) ist.

2. Aktivierungs-/Einzugsmechanismus nach Anspruch 1, wobei ein distaler Abschnitt (14; 24) des Drückers (10; 20) und ein proximaler Abschnitt (19; 29) des Lanzettenhalters (15; 25) komplementäre Querschnitte aufweisen und eine kombinierte Querschnittsfläche kleiner als die Querschnittsfläche des Gehäuses aufweisen, so dass der distale Abschnitt (14; 24) des Drückers (10; 20) und der proximale Abschnitt (19; 29) des Lanzettenhalters (15; 25) in Bezug zueinander frei beweglich sind, wenn das distale Ende (13; 23) des Drückers (10; 20) vom proximalen Ende (18; 28) des Lanzettenhalters (15; 25) gelöst ist.

3. Aktivierungs-/Einzugsmechanismus nach Anspruch 2, wobei der Drücker (10) und der Lanzettenhalter (15) in einem Winkel ungleich null zueinander angeordnet sind, so dass sich das distale Ende (13) des Drückers (10) in Bezug auf das proximale Ende (18) des Lanzettenhalters (15) seitlich bewegt, wenn das erste Vorspannelement (17) den Drücker (10) distal verschiebt, bis sie sich voneinander lösen.

4. Aktivierungs-/Einzugsmechanismus nach Anspruch 2, wobei der Drücker (20) eine äußere spiralförmige Nut oder Rippe (21) umfasst, die derart angeordnet ist, dass sie mit einem im Gehäuse angeordneten Führungsvorsprung (22) zusammenwirkt, um den Drücker (20) zu drehen, wenn das erste Vorspannelement den Drücker (20) distal verschiebt, so dass sich das distale Ende (23) des Drückers (20) in Bezug auf das proximale Ende (28) des Lanzettenhalters (25) dreht, bis sie sich voneinander lösen.

5. Aktivierungs-/Einzugsmechanismus nach Anspruch 1, wobei der Drücker (30; 40) mindestens einen Drückerarm (31; 41) umfasst, der sich in einer distalen Richtung erstreckt, wobei ein distales Ende (33; 43) des mindestens einen Drückerarms (31; 41) eine Eingriffsfläche (32; 42), die zum Eingriff mit dem proximalen Ende (38; 48) des Lanzettenhalters (35; 45) angeordnet ist, und eine Nockenfläche (34; 44) umfasst, wobei die Nockenfläche (34; 44) angeordnet ist, um mit einem Widerlager (36; 46) im Gehäuse in Kontakt zu kommen, das in einer Position angeordnet ist, die der vorbestimmten Länge von der Ausgangsposition des Drückers (30; 40) entspricht, und angepasst ist, den mindestens einen Drückerarm (31; 41) seitlich abzulenken, um den mindestens einen Drückerarm (31; 41) vom proximalen Ende (38; 48) des Lanzettenhalters (35; 45) zu lösen, wenn der Drücker (30; 40) in distaler Richtung gegen das Widerlager (36; 46) gedrückt wird.

6. Aktivierungs-/Einzugsmechanismus nach Anspruch 5, wobei der Drücker (40) zwei Drückerarme (41) umfasst.

7. Aktivierungs-/Einzugsmechanismus nach Anspruch 5 oder 6, der weiter eine Sicherheitsanordnung umfasst, die einen Lanzettenschutz (67) umfasst, der einstückig mit einem distalen Ende (49) des Lanzettenhalters (45) mittels eines zerbrechlichen Abschnitts ausgebildet ist, der dazu angepasst ist, zu brechen, wenn der Lanzettenschutz (67) in Bezug auf den Lanzettenhalter (45) verschoben wird.

8. Aktivierungs-/Einzugsmechanismus nach Anspruch 7, wobei der Lanzettenhalter (45) weiter einen oder mehrere Vorsprünge (62) umfasst, die derart angeordnet sind, dass sie mit entsprechenden Aussparungen (64) im Gehäuse (65) zusammenwirken, die eine Drehung des Lanzettenhalters (45) innerhalb eines vorbestimmten Winkelbereichs ermöglichen.

9. Aktivierungs-/Einzugsmechanismus nach Anspruch 7 oder 8, wobei die Sicherheitsanordnung weiter eine Führungsstange (60) umfasst, die starr mit einem proximalen Ende (48) des Lanzettenhalters (45) verbunden oder einstückig damit ausgebildet ist, wobei der Drücker (40) ein Durchgangsloch (61) umfasst, das an die Form der Führungsstange (60) angepasst ist, so dass Drehung des Lanzettenhalters (45) um einen vorbestimmten Winkel in Bezug auf den Drücker (40) die Führungsstange (60) mit dem Loch (61) in Ausrichtung bringt, um dem Drücker (40) zu ermöglichen, entlang der Führungsstange (60) distal verschoben zu werden.

10. Aktivierungs-/Einzugsmechanismus nach einem der vorstehenden Ansprüche, wobei das erste Vorspannelement (11; 66; 51) und/oder das zweite Vorspannelement (17; 47; 57) eine elastische Feder umfassen.

11. Aktivierungs-/Einzugsmechanismus nach einem der vorstehenden Ansprüche, der weiter ein lösbares Verriegelungselement (9; 63; 56) umfasst, das so angeordnet ist, dass es den Drücker (10; 40; 50) und das erste Vorspannelement (11; 66; 51) in einem vorgespannten Zustand hält.

12. Aktivierungs-/Einzugsmechanismus nach einem der vorstehenden Ansprüche, wobei der Drücker (10; 20; 30; 40; 50) in einem Anfangsstadium in einem Abstand vom Lanzettenhalter (15; 25; 35; 45; 55) angeordnet ist, so dass das distale Ende des Drückers (10; 20; 30; 40; 50) vom proximalen Ende (18; 28; 38; 48; 58) des Lanzettenhalters (15; 25; 35; 45; 55) getrennt ist.

13. Lanzettiervorrichtung, die einen Aktivierungs-/Einzugsmechanismus nach einem der vorstehenden Ansprüche umfasst.

## Revendications

1. Mécanisme d'activation/de rétraction pour un dispositif autopiqueur comprenant :
- un poussoir (10 ; 20 ; 30 ; 40 ; 50) comprenant un premier élément de sollicitation (11 ; 66 ; 51) agencé pour déplacer le poussoir (10 ; 20 ; 30 ; 40 ; 50) dans une direction distale ; et
- un porte-lancette (15 ; 25 ; 35 ; 45 ; 55) comprenant une lancette (16) pour pénétrer la peau d'un sujet, et un second élément de sollicitation (17 ; 47 ; 57) agencé pour déplacer le porte-lancette (15 ; 25 ; 35 ; 45 ; 55) dans une direction proximale ;
dans lequel le porte-lancette (15 ; 25 ; 35 ; 45 ; 55) et le poussoir (10 ; 20 ; 30 ; 40 ; 50) sont agencés dans un boîtier (12 ; 42) du dispositif autopiqueur de telle sorte qu'une extrémité distale (13 ; 23 ; 33 ; 43 ; 53) du poussoir (10 ; 20 ; 30 ; 40 ; 50) engage une extrémité proximale (18 ; 28 ; 38 ; 48 ; 58) du porte-lancette (15 ; 25 ; 35 ; 45 ; 55) pour pousser le porte-lancette (15 ; 25 ; 35 ; 45 ; 55) dans une direction distale lorsque le premier élément de sollicitation (11 ; 66 ; 51) déplace le poussoir (10 ; 20 ; 30 ; 40 ; 50) de manière distale,
dans lequel les directions de déplacement du poussoir (10 ; 20 ; 30 ; 40 ; 50) et du porte-lancette (15 ; 25 ; 35 ; 45 ; 55) sont sensiblement parallèles à une extension longitudinale du boîtier (12 ; 42),
**caractérisé en ce que** l'extrémité distale (13 ; 23 ; 33 ; 43 ; 53) du poussoir (10 ; 20 ; 30 ; 40 ; 50) et l'extrémité proximale (18 ; 28 ; 38 ; 48 ; 58) du porte-lancette (15 ; 25 ; 35 ; 45 ; 55) sont agencées pour se désengager l'une de l'autre lorsque le poussoir (10 ; 20 ; 30 ; 40 ; 50) a été déplacé de manière distale d'une longueur prédéterminée par rapport à sa position initiale, selon lequel le porte-lancette (15 ; 25 ; 35 ; 45 ; 55) est autorisé à revenir à sa position initiale sous l'influence du second élément de sollicitation (17 ; 47 ; 57), et
une surface d'engagement de l'extrémité proximale (18 ; 28 ; 38 ; 48 ; 58) du porte-lancette (15 ; 25 ; 35 ; 45 ; 55) est perpendiculaire à la direction de déplacement du porte-lancette (15 ; 25 ; 35 ; 45 ; 55).

2. Mécanisme d'activation/de rétraction selon la revendication 1, dans lequel une partie distale (14 ; 24) du poussoir (10 ; 20) et une partie proximale (19 ; 29) du porte-lancette (15 ; 25) présentent des sections transversales complémentaires et présentent une zone de section transversale combinée inférieure à la zone de section transversale du boîtier de sorte que la partie distale (14 ; 24) du poussoir (10 ; 20) et la partie proximale (19 ; 29) du porte-lancette (15 ; 25) soient libres de se déplacer l'une par rapport à l'autre lorsque l'extrémité distale (13 ; 23) du poussoir (10 ; 20) est dégagée de l'extrémité proximale (18 ; 28) du porte-lancette (15 ; 25).

3. Mécanisme d'activation/de rétraction selon la revendication 2, dans lequel le poussoir (10) et le porte-lancette (15) sont agencés à un angle non nul l'un par rapport à l'autre de sorte que l'extrémité distale (13) du poussoir (10) se déplace latéralement par rapport à l'extrémité proximale (18) du porte-lancette (15) lorsque le premier élément de sollicitation (17) déplace le poussoir (10) de manière distale jusqu'à ce qu'elles se désengagent l'une de l'autre.

4. Mécanisme d'activation/de rétraction selon la revendication 2, dans lequel le poussoir (20) comprend une rainure ou arête hélicoïdale externe (21) agencée pour coopérer avec une saillie de guidage (22) agencée dans le boîtier pour faire tourner le poussoir (20) lorsque le premier élément de sollicitation déplace le poussoir (20) de manière distale, de sorte que l'extrémité distale (23) du poussoir (20) tourne par rapport à l'extrémité proximale (28) du porte-lancette (25) jusqu'à ce qu'elles se désengagent l'une de l'autre.

5. Mécanisme d'activation/de rétraction selon la revendication 1, dans lequel le poussoir (30 ; 40) comprend au moins un bras poussoir (31 ; 41) s'étendant dans une direction distale, dans lequel une extrémité distale (33 ; 43) du au moins un bras poussoir (31 ; 41) comprend une surface d'engagement (32 ; 42) agencée pour engager l'extrémité proximale (38 ; 48) du porte-lancette (35 ; 45) et une surface de came (34 ; 44), dans lequel la surface de came (34 ; 44) est agencée pour venir en contact avec une butée (36 ; 46) dans le boîtier agencée à une position correspondant à la longueur prédéterminée depuis la position initiale du poussoir (30 ; 40) et adaptée pour dévier le au moins un bras poussoir (31 ; 41) latéralement pour désengager le au moins un bras poussoir (31 ; 41) de l'extrémité proximale (38 ; 48) du porte-lancette (35 ; 45) lorsque le poussoir (30 ; 40) est poussé contre la butée (36 ; 46) dans la direction distale.

6. Mécanisme d'activation/de rétraction selon la revendication 5, dans lequel le poussoir (40) comprend deux bras poussoirs (41).

7. Mécanisme d'activation/de rétraction selon la revendication 5 ou 6, comprenant en outre un ensemble de sécurité comprenant un protège-lancette (67) formé d'un seul tenant avec une extrémité distale (49) du porte-lancette (45) au moyen d'une partie frangible adaptée pour se rompre lorsque le protège-lancette (67) est déplacé par rapport au porte-lancette (45).

8. Mécanisme d'activation/de rétraction selon la revendication 7, dans lequel le porte-lancette (45) comprend en outre une ou plusieurs saillies (62) agencées pour coopérer avec des évidements correspondants (64) dans le boîtier (65) qui permettent la rotation du porte-lancette (45) dans une plage angulaire prédéterminée.

9. Mécanisme d'activation/de rétraction selon la revendication 7 ou 8, dans lequel l'ensemble de sécurité comprend en outre une tige de guidage (60) reliée rigidement à ou formée intégralement avec une extrémité proximale (48) du porte-lancette (45), dans lequel le poussoir (40) comprend un trou traversant (61) adapté à la forme de la tige de guidage (60) de sorte que la rotation du porte-lancette (45) d'un angle prédéterminé par rapport au poussoir (40) amène la tige de guidage (60) en alignement avec le trou (61) pour permettre au poussoir (40) de se déplacer de manière distale le long de la tige de guidage (60).

10. Mécanisme d'activation/de rétraction selon l'une quelconque des revendications précédentes, dans lequel le premier élément de sollicitation (11 ; 66 ; 51) et/ou le second élément de sollicitation (17 ; 47 ; 57) comprennent un ressort élastique.

11. Mécanisme d'activation/de rétraction selon l'une quelconque des revendications précédentes, comprenant en outre un élément de verrouillage libérable (9 ; 63 ; 56) agencé pour retenir le poussoir (10 ; 40 ; 50) et le premier élément de sollicitation (11 ; 66 ; 51) dans un état précontraint.

12. Mécanisme d'activation/de rétraction selon l'une quelconque des revendications précédentes, dans lequel le poussoir (10 ; 20 ; 30 ; 40 ; 50) est agencé à distance du porte-lancette (15 ; 25 ; 35 ; 45 ; 55) à un stade initial de sorte que l'extrémité distale du poussoir (10 ; 20 ; 30 ; 40 ; 50) soit séparée de l'extrémité proximale (18 ; 28 ; 38 ; 48 ; 58) du porte-lancette (15 ; 25 ; 35 ; 45 ; 55).

13. Dispositif autopiqueur comprenant un mécanisme d'activation/de rétraction selon l'une quelconque des revendications précédentes.
